# EUROPEAN PATENT APPLICATION

(11) **EP 3 437 723 A1**
(43) Date of publication of application: **06.02.2019**
(21) Application number: 17775546.9
(22) Date of filing: 31.03.2017
(51) Int. Cl.: B01D 63/02, B01D 63/00

(54) **HOLLOW FIBER MEMBRANE MODULE AND METHOD FOR MANUFACTURING HOLLOW FIBER MEMBRANE MODULE**

(30) Priority: 31.03.2016 JP 2016073294
(71) Applicant: Asahi Kasei Medical Co., Ltd., Chiyoda-ku Tokyo 101-8101 (JP)
(72) Inventor: KOYANO, Toshihiro, Tokyo 101-8101 (JP); ASATSUMA, Keiichi, Tokyo 101-8101 (JP); KOIZUMI, Toshinori, Tokyo 101-8101 (JP)
(74) Representative: dompatent von Kreisler Selting Werner - Partnerschaft von Patent- und Rechtsanwälten mbB
(86) International application number: PCT/JP2017/013637
(87) International publication number: WO 2017/171015

(57) **Abstract**

A hollow fiber membrane module 1 includes: a tubular container 2 accommodating a hollow fiber membrane bundle 3; headers 5, and potted parts 4. The header 5 includes a header protrusion 22 that protrudes in an annular shape toward the tubular container 2. The tubular container 2 includes double annular protrusions, which are an inner protrusion 50 and an outer protrusion 51 protruding toward the header 5. The header 5 and the tubular container 2 are welded together by ultrasonic welding, with the header protrusion 22 being inserted between the inner protrusion 50 and the outer protrusion 51 of the tubular container 2. The header protrusion 22 and the inner protrusion 50 of the tubular container 2 are welded together at least in one or more regions, and the header protrusion 22 and the outer protrusion 51 of the tubular container 2 are welded together at least in one or more regions.

## Description

### Cross Reference to Related Applications

**The** present application is based on Japanese Patent Application No. 2016-073294 filed on March 31, 2016, the contents of which are incorporated herein by reference.

### Technical Field

The present invention relates to a hollow fiber membrane module and a method of producing the hollow fiber membrane module.

### Background Art

Hollow fiber membrane modules are used as a blood purifier or a component separator, for instance, in blood purification processes in extracorporeal circulation systems such as hemodialysis, hemodiafiltration, hemofiltration, and plasma separation, processing of coelomic fluids such as ascitic fluid, removal of viruses from blood products, and so on. A hollow fiber membrane module commonly includes a tube-like tubular container accommodating a hollow fiber membrane bundle therein and having two ports for letting a fluid to flow in and out (inlet port and outlet port) in a side face, and a header provided at each of both ends of this tubular container and having a fluid inlet/outlet port. The fluid inlet/outlet ports of the headers communicate with inner regions of hollow fiber membranes, while the lateral ports of the tubular container communicate with outer regions of the hollow fiber membranes. In hemodialysis that uses a hollow fiber membrane module, for example, blood is introduced from one header, passed through the inner regions of the hollow fiber membranes, and discharged from the other header, while dialysate is introduced from one lateral port of the tubular container and discharged from the other port, so that unnecessary substances are separated from the blood and transferred to the dialysate through the hollow fiber membranes.

In a production step of the hollow fiber membrane module described above, the above-mentioned headers are fitted to ends of the tubular container such as to cover the outer sides of the ends of the tubular container, and welded to the ends of the tubular container by ultrasonic welding (see Patent Document 1).

### Citation List

### Patent Document

Patent Document 1: WO 2013/146663

### Summary

### Technical Problem

The use of the hollow fiber membrane module described above involves concerns about possible separation of the headers and the tubular container, or partial failure of welds between the headers and the tubular container in conditions where the hollow fiber membrane module is subjected to internal pressure (conditions under internal pressure). The weld strength against pressure between the headers and the tubular container therefore need to be enhanced in order to prevent leakage of process fluids out of the system (module).

The present application was filed in view of this issue and its object is to increase the pressure capacity of the hollow fiber membrane module.

### Solution to Problem

Through vigorous research, the present inventors found out that the pressure capacity of hollow fiber membrane modules is increased by welding the header and the tubular container together by ultrasonic welding, with a header protrusion being inserted between an inner protrusion and an outer protrusion of the tubular container, wherein the header protrusion and the inner protrusion of the tubular container are welded together at at least one or more regions, and the header protrusion and the outer protrusion of the tubular container are welded together at at least one or more regions, which eventually led to the completion of the present invention.

Namely, the present invention includes the following aspects:
(1) A hollow fiber membrane module including: a tubular container accommodating a hollow fiber membrane bundle; a header provided to each of both end parts of the tubular container and having an outlet/inlet port of a fluid; and a potted part retaining fixedly the hollow fiber membrane bundle embedded therein at each of the both end parts of the tubular container, the header having a header protrusion protruding toward the tubular container, the tubular container having double protrusions, which are an inner protrusion and an outer protrusion protruding toward the header, the header and the tubular container being welded together by ultrasonic welding, with the header protrusion being inserted between the inner protrusion and the outer protrusion of the tubular container, and the header protrusion and the inner protrusion of the tubular container being welded together at least in one or more regions, and the header protrusion and the outer protrusion of the tubular container being welded together at least in one or more regions.
(2) The hollow fiber membrane module according to (1), wherein at least one portion of a weld between the header protrusion and the outer protrusion of the tubular container, and at least one portion of a weld between the header protrusion and the inner protrusion of the tubular container, are located substantially at the same position in a longitudinal direction of the tubular container.
(3) The hollow fiber membrane module according to (1) or (2), wherein a weld is formed between the header protrusion and the inner protrusion of the tubular container at a plurality of different locations in the longitudinal direction of the tubular container, and a region where the header and the tubular container do not contact each other exists between the welds at different locations.
(4) The hollow fiber membrane module according to any one of (1) to (3), wherein a region where the header and the tubular container do not contact each other exists between a weld between the header protrusion and the inner protrusion of the tubular container and a weld between the header protrusion and the outer protrusion of the tubular container.
(5) The hollow fiber membrane module according to any one of (1) to (4), wherein at least a portion of the weld between the header protrusion and the inner protrusion of the tubular container, or, at least a portion of the weld between the header protrusion and the outer protrusion of the tubular container, is circumferentially continuously formed all around the header.
(6) The hollow fiber membrane module according to any one of (1) to (5), wherein the inner protrusion of the tubular container and the header protrusion are welded together at two regions, while the outer protrusion of the tubular container and the header protrusion are welded together at one region.
(7) The hollow fiber membrane module according to any one of (1) to (6), wherein an end face of the potted part at each of the both ends to be formed as a potted part sectional surface by forming radially cutting the potted part at each of the both ends, and the potted part sectional surface is in contact with an inner side face of the header.
(8) The hollow fiber membrane module according to any one of (1) to (7), wherein the header and the tubular container are made of polypropylene resin.
(9) A method of producing hollow fiber membrane modules including a tubular container accommodating a hollow fiber membrane bundle, a header provided to each of both end parts of the tubular container and having an outlet/inlet port of a fluid, and a potted part retaining fixedly the hollow fiber membrane bundle embedded therein at each of the both end parts of the tubular container, the method including the steps of: forming the header such that the header has a header protrusion protruding toward the tubular container having double protrusions, which are an inner protrusion and an outer protrusion protruding toward the header; and welding the header and the tubular container together by ultrasonic welding, with the header protrusion being inserted between the inner protrusion of the tubular container and the outer protrusion of the tubular container, wherein in the welding step, the header protrusion and the inner protrusion of the tubular container are welded together at least in one or more regions, and the header protrusion and the outer protrusion of the tubular container are welded together at least in one or more regions. Advantageous Effects of Invention

According to the present invention, the pressure capacity of the hollow fiber membrane module can be increased, so that possibilities of separation of the headers and the tubular container, and partial failure of welds, are reduced, whereby leakage of process fluids out of the system can be prevented.

### Brief Description of Drawings

Fig. 1 is a schematic diagram of a longitudinal cross section illustrating one configuration example of a hollow fiber membrane module.
Fig. 2 is an enlarged cross-sectional view illustrating one configuration example of a joint part between a header and a tubular container.
Fig. 3 is an A-A cross-sectional view of the joint part between the header and the tubular container.
Fig. 4 is a B-B cross-sectional view of the joint part between the header and the tubular container.
Fig. 5 is a cross-sectional view illustrating how the header and the tubular container are ultrasonically welded together.
Fig. 6 is an enlarged cross-sectional view indicating directions of forces acting on the joint part between the header and the tubular container.
Fig. 7 is an enlarged cross-sectional view illustrating one configuration example of a joint part between the header and the tubular container wherein another examples of welds are shown.
Fig. 8 is an enlarged cross-sectional view illustrating one configuration example of a joint part between the header and the tubular container wherein another examples of welds are shown.
Fig. 9 is a B-B cross-sectional view of the joint part between the header and the tubular container that are welded together intermittently.
Fig. 10 is a table showing the results in examples.

### Description of Embodiments

Hereinafter, a preferred embodiment of the present invention will be described with reference to the drawings. The same elements are provided with the same reference numerals, and redundant description thereof will be omitted. Positional relationships such as upper and lower, or left and right, etc., shall be based on the positional relationships indicated in the drawings unless otherwise specified. The dimensional ratios in the drawings shall not be limited to the illustrated ratios. The following embodiment is described for illustrative purposes only and the present invention shall not be limited to this embodiment.

### <Configuration of hollow fiber membrane module>

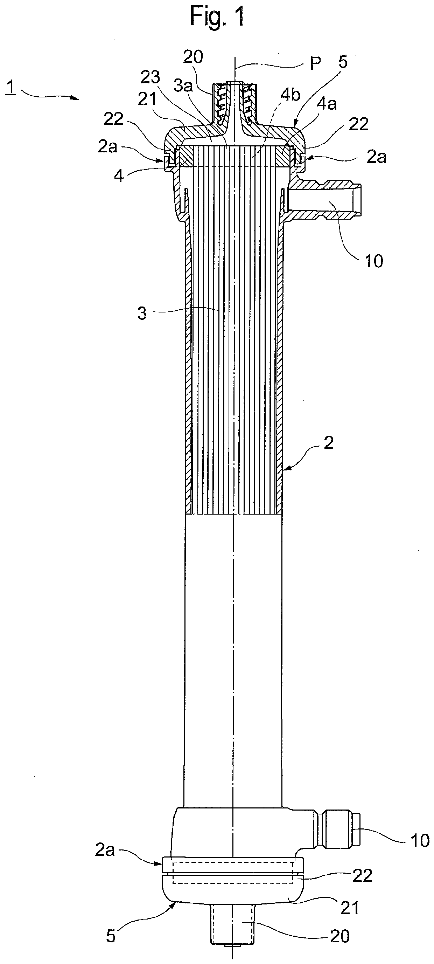

First, the configuration of the hollow fiber membrane module according to this embodiment will be described. Fig. 1 is a schematic cross-sectional view illustrating one configuration example of a hollow fiber membrane module 1.

**The** hollow fiber membrane module 1 includes a tubular container 2, a hollow fiber membrane bundle 3, potted parts 4, headers 5, and so on.

The tubular container 2 is cylindrical and open at both end parts 2a in a longitudinal direction (along the center axis P of the cylinder). The hollow fiber membrane bundle 3 is accommodated inside the tubular container 2. Two ports 10, for example, that are an inlet and an outlet of a fluid, are formed in a side face of the tubular container 2.

The hollow fiber membrane bundle 3 is a bundle of a large number of hollow fiber membranes and accommodated inside the tubular container 2 along the longitudinal direction. The hollow fiber membrane bundle 3 functions as a separation membrane, and can separate a component to be separated from the fluid between inner regions and outer regions of respective hollow fiber membranes.

The potted parts 4 are made of potting resin. Both end parts 3a of the hollow fiber membrane bundle 3 are embedded in the potted parts inside both end parts 2a of the tubular container 2, whereby the hollow fiber membrane bundle 3 is fixed to both end parts 2a of the tubular container 2. The potted part 4 includes an outer circumferential portion 4a solely made of the potting resin, and an inner portion 4b where gaps between the hollow fiber membranes of the hollow fiber membrane bundle 3 are filled with the potting resin. The potting resin may be, for example, polyurethane resin, epoxy resin, silicone resin and the like, but is not limited particularly to these resins.

The header 5 is provided to both end parts 2a of the tubular container 2 as a lid member for the opening of both end parts 2a. The header 5 includes a tube-like nozzle part 20 as an inlet and outlet port for a fluid along the center axis P, a platelike top plate 21 extending radially from the nozzle part 20, and a header protrusion 22 as a side wall protruding from the outer peripheral edge of the top plate 21 toward the tubular container 2.

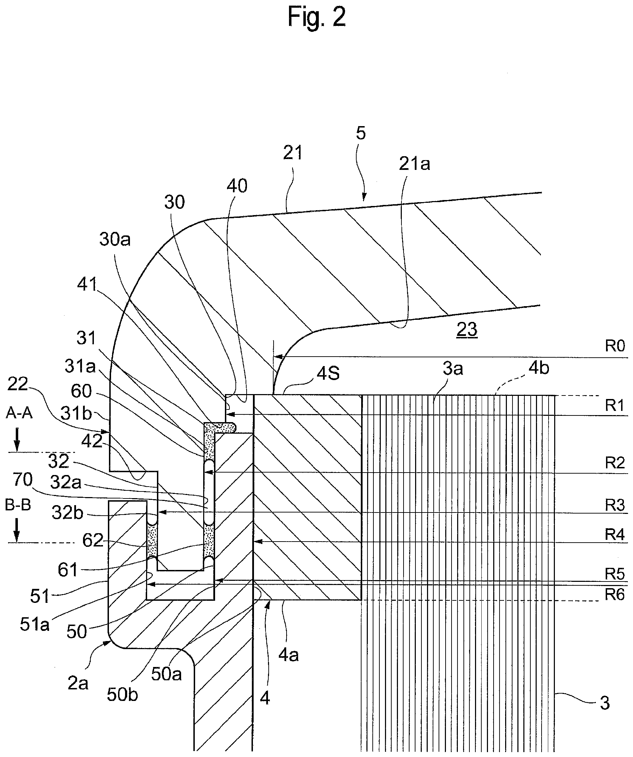

The nozzle part 20 has a thread configuration for allowing connection of an outer tube. As shown in Fig. 1 and Fig. 2, the top plate 21 has an inner face 21a opposite the end part 3a of the hollow fiber membrane bundle 3, with a diameter gradually increasing from the nozzle part 20 toward the end part 2a of the tubular container 2. There is formed a space 23 between the inner face 21a of the header 5 and the end part 3a of the hollow fiber membrane bundle 3 for the fluid flowing in through the nozzle part 20 or the fluid flowing out from the nozzle part 20 to pass through.

### <Header protrusion>

The header protrusion 22 is cylindrical having the center axis P as its axial center. The header protrusion 22 includes, as shown in Fig. 2, a base part 30, a middle part 31, and a distal end part 32 in this order from the top plate 21 toward the tubular container 2. The base part 30, middle part 31, and distal end part 32 are each different in radial thickness; the base part 30 has the largest thickness, the middle part 31 has the second largest thickness, and the distal end part 32 has the smallest thickness.

The base part 30 is a portion continuous from the top plate 21 and has a first inner circumferential surface 30a with a constant inner diameter R1, for example. The inner diameter R1 of the first inner circumferential surface 30a is larger than the inner diameter R0 at the lower end of the inner face 21a of the top plate 21 (largest diameter part). An annular flat surface (step) 40 perpendicular to the center axis P is formed between the inner face 21a of the top plate 21 and the first inner circumferential surface 30a of the base part 30.

The middle part 31 has a second inner circumferential surface 31a with a constant inner diameter R2 that is larger than the inner diameter R1. The distal end part 32 has a third inner circumferential surface 32a with the same inner diameter R2 as that of the second inner circumferential surface 31a, and an outer circumferential surface 32b with a constant outer diameter R3 that is smaller than the outer diameter of the outer circumferential surface 31b of the middle part 31. An annular flat surface (step) 41 perpendicular to the center axis P is formed between the first inner circumferential surface 30a of the base part 30 and the second inner circumferential surface 31a of the middle part 31. An annular flat surface (step) 42 perpendicular to the center axis P is formed between the outer circumferential surface 31b of the middle part 31 and the outer circumferential surface 32b of the distal end part 32.

### <Tubular container (inner protrusion/outer protrusion)>

The end part 2a of the tubular container 2 has a so-called bifurcated structure. The end part 2a has cylindrical double protrusions, which are an inner protrusion 50 and an outer protrusion 51 protruding toward the header 5. The inner protrusion 50 and outer protrusion 51 each have a cylindrical shape having the center axis P as their axial center and are arranged concentrically. The inner protrusion 50 is longer than the outer protrusion 51 and protrudes toward the header 5 (outward in the direction along the center axis P). The inner circumferential surface 50a of the inner protrusion 50 forms the inner circumferential surface of the tubular container 2.

The inner circumferential surface 50a of the inner protrusion 50 has an inner diameter R4 that is larger than the header inner diameter R0 and smaller than the header inner diameter R1. The outer circumferential surface 50b of the inner protrusion 50 has an outer diameter R5 that is larger than the header inner diameter R1 and smaller than the header inner diameter R2. The inner circumferential surface 51a of the outer protrusion 51 has an inner diameter R6 that is larger than the header outer diameter R3.

### <Joint structure between the header and tubular container>

With the header protrusion 22 being inserted between the inner protrusion 50 and the outer protrusion 51 of the tubular container 2, the header 5 and tubular container 2 are welded together by ultrasonic welding. The inner protrusion 50 faces the middle part 31 and distal end part 32 of the header protrusion 22, and the outer protrusion 51 faces the distal end part 32.

The inner protrusion 50 and header protrusion 22 are welded together at a region between the vicinity of the distal end of the inner protrusion 50 and the middle part 31 of the header protrusion 22 (first weld 60), and a region between the inner protrusion 50 and the distal end part 32 (second weld 61).

The outer protrusion 51 and the header protrusion 22 are welded together at a region between the outer protrusion 51 and the distal end part 32 (third weld 62).

### <First weld>

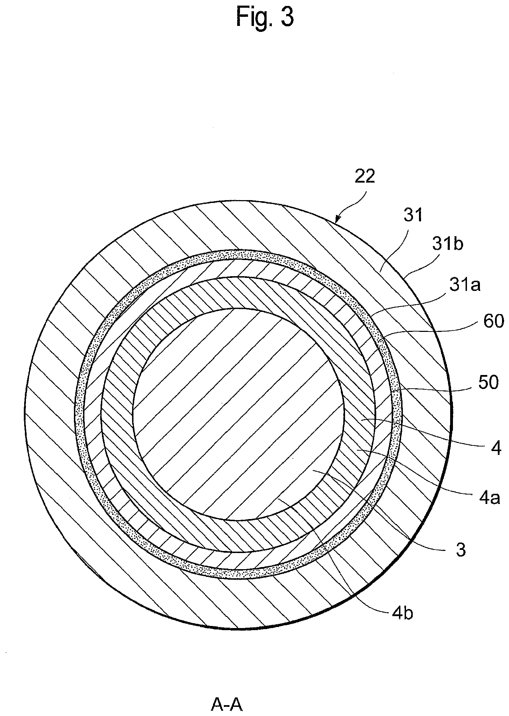

The first weld 60 is formed to extend from an end face (upper end face in Fig. 2), for example, of the inner protrusion 50 over to the outer circumferential surface 50b. While Fig. 2 clearly shows boundaries between the welds 60 to 62 for convenience of illustration, the actual boundaries are indistinct in welding where contacting parts fuse and join together, and vary in each welding. The first weld 60 may be formed circumferentially continuously all around the header protrusion 22 as shown in Fig. 3, or may be formed non-continuously (intermittently). The first weld 60 continuously formed all around would be preferable in the respect that the path (region) the process fluid may enter as it tries to leak out of the system can be made as short as possible, because, should a process fluid such as blood goes beyond the contacting parts of the header 5 and the potted part 4, the fluid can be stopped by the nearest weld 60.

### <Second weld>

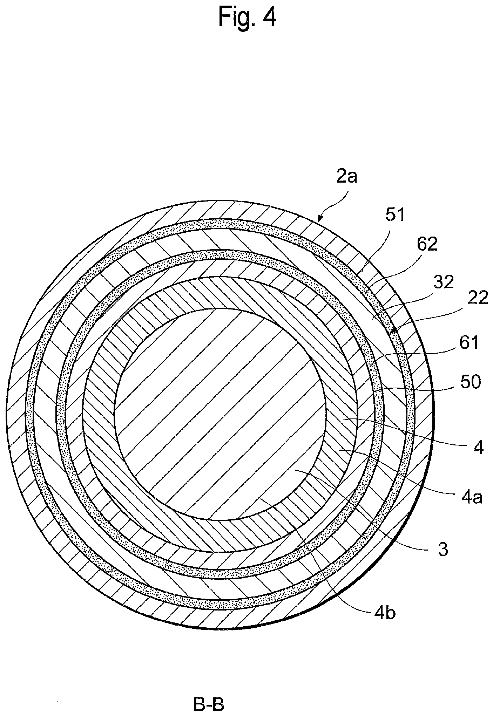

As shown in Fig. 2, the second weld 61 is formed near the tip of the distal end part 32 of the header protrusion 22, for example (located closer to the distal end than the center in the longitudinal direction of the distal end part 32). The second weld 61 is separated from the first weld 60, and there is a space 70 where the header protrusion 22 does not contact the inner protrusion 50 formed between the second weld 61 and the first weld 60. The second weld 61 may be formed circumferentially continuously all around the header protrusion 22 as shown in Fig. 4, or may be formed non-continuously (intermittently). If the second weld 61 is continuously formed, it would be preferable in respect of prevention of leakage of the process fluid out of the system. If the second weld 61 is formed non-continuously, energy required for the welding of the header 5 and the tubular container 2 can be reduced, so that it would be preferable in respect of improvement of production efficiency.

### <Third weld>

The third weld 62 is formed near the tip of the distal end part 32 of the header protrusion 22, for example (located closer to the distal end than the center in the longitudinal direction of the distal end part 32). The third weld 62 may be formed circumferentially continuously all around the header protrusion 22 as shown in Fig. 4, or may be formed non-continuously (intermittently).

As shown in Fig. 2, the second weld 61 and the third weld 62 are formed substantially at the same position in the direction of the center axis P (longitudinal direction), for example. Here, "substantially the same" means that at least parts of the second weld 61 and the third weld 62 are located in the same position along the direction of the center axis P.

### <Arrangement of potted part>

As shown in Fig. 2, an end face of the potted parts 4 is formed as a potted part sectional surface 4S by cutting the potted parts at both ends radially. The potted part 4 protrudes outward (toward the header 5) in the direction of the center axis P more than the inner protrusion 50 of the tubular container 2. The potted part sectional surface 4S is in contact with the flat surface 40 of the inner face of the header 5 under a predetermined pressure.

Materials for the tubular container 2 and headers 5 are not limited particularly and may be selected from various thermoplastic resins. Examples of crystalline resins include ethylene α-olefin copolymers, polyethylene resins such as low-density polyethylene and high-density polyethylene, propylene homopolymer, and polypropylene resins such as propylene-ethylene copolymers or copolymers of propylene, ethylene, and other α-olefins, while examples of amorphous resins include resins represented by polyester, polycarbonate, polystyrene, styrenebutadiene copolymer (SBS), acrylonitrile butadiene styrene (ABS) copolymer, all of which may be used alone or as a mixture.

Among the resins listed above, polypropylene resin is preferable in this embodiment, and random copolymers of propylene and ethylene are particularly preferable in respect of rigidity and heat resistance. A random copolymer of propylene and ethylene with a content of ethylene adjusted to 1 to 8 wt% is even more preferable.

### <Production method of hollow fiber membrane module>

First, the hollow fiber membrane bundle 3 is prepared to have a larger length than would eventually be necessary, and accommodated in the tubular container 2. Next, the potted parts 4 are formed inside the tubular container 2. The potted parts 4 retain the hollow fiber membrane bundle 3 to the inner circumferential surface 50a of the tubular container 2, with both end parts 3a of the hollow fiber membrane bundle 3 embedded therein. Next, unnecessary parts of the potted parts 4 (both end parts 3a of the hollow fiber membrane bundle 3) are cut in a cross section perpendicular to the center axis P so that the potted part sectional surfaces 4S are formed.

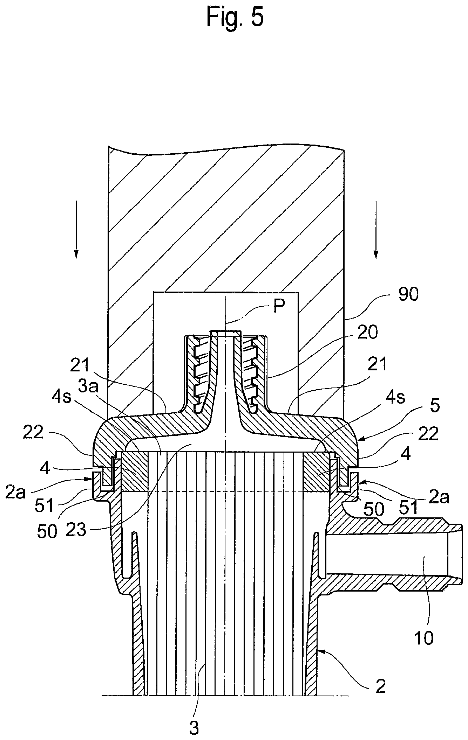

Next, the headers 5 are welded to the tubular container 2. The header protrusion 22 of the header 5 is inserted between the inner protrusion 50 and the outer protrusion 51 of the tubular container 2 as shown in Fig. 5, for example, and an ultrasonic horn 90 moves toward the top plate 21 of the header 5 to apply ultrasonic vibration while pressing the header 5 from outside toward the tubular container 2 in the direction of the center axis P. The applied ultrasonic vibration generates friction heat in regions where the header 5 and tubular container 2 contact each other, whereby the first weld 60, second weld 61, and third weld 62 are formed. The potted part sectional surface 4S is then abutted to the flat surface 40 of the inner face of the header 5. At least one of the header protrusion 22, or the inner protrusion 50 and outer protrusion 51 is provided with an interference configuration (weld joint design) in portions that will become the welds 60, 61, and 62, which causes the header 5 and the tubular container 2 to contact and interfere with each other during the welding, so that they are welded together at the locations of the welds 60, 61, and 62. The header 5 and the tubular container 2 are thus welded together at three points and the finished hollow fiber membrane module 1 shown in Fig. 1 is obtained.

### <Weld joint design>

The weld joint design is not limited particularly, but a butt joint design or a shear joint design may be selected. A butt joint has a triangular rib known as an energy director that causes the expanding and contracting motion by the ultrasonic vibration to concentrate in the triangular rib, which offers the advantage of being able to heat the resin up to its melting temperature in a very short time, and therefore can be used preferably for the first weld 60. The shear joint is beneficial in that bubbles hardly form in the weld surfaces and the welds have excellent sealing properties against liquids and gasses, because in the shear joint the contact surface and the vibration direction come close in the same direction as the direction of the vertical vibration applied by ultrasound, and therefore can be used preferably for the first, second, and third welds 60, 61, and 62.

According to this embodiment, the header 5 and the tubular container 2 are welded together by ultrasonic welding, with the header protrusion 22 being inserted between the inner protrusion 50 and the outer protrusion 51 of the tubular container 2, at least in one or more regions (welds 60 and 61) of the header protrusion 22 and the inner protrusion 50, and at least in one or more regions (welds 62) of the header protrusion 22 and the outer protrusion 51. Consequently, the header 5 can hardly be separated from the tubular container 2, and the pressure capacity of the hollow fiber membrane module 1 can be enhanced. Moreover, the header 5 and the tubular container 2 are directly welded together without any component therebetween, so that the header 5 and the tubular container 2 are firmly joined. The production efficiency is improved, and the cost can be reduced.

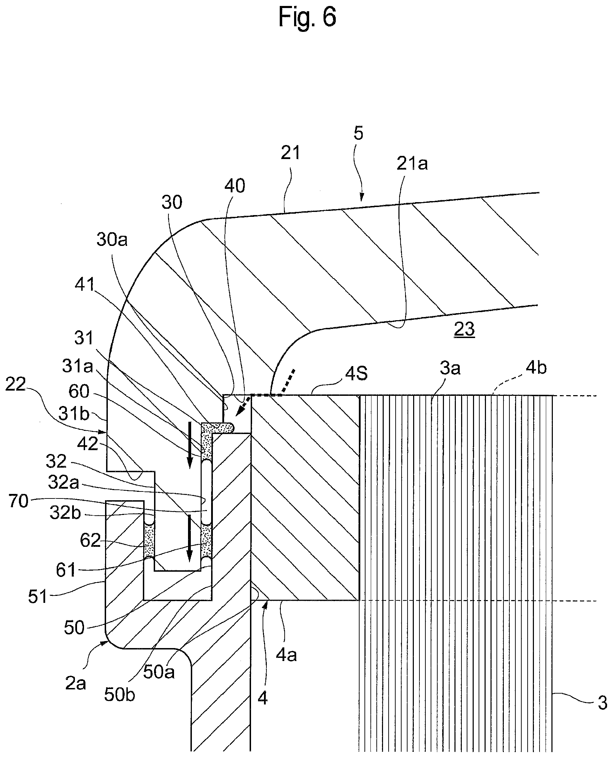

Under the internal pressure of the process fluid in the hollow fiber membrane module 1, stress is applied to the weld points between the header 5 and the tubular container 2 that acts to pull the header 5 apart from the tubular container 2. In this embodiment, they are welded together at least in one or more regions (welds 60 and 61) of the header protrusion 22 and the inner protrusion 50, and at least in one or more regions (welds 62) of the header protrusion 22 and the outer protrusion 51, so that this stress can be dispersed, and thus the pressure capacity is improved. If the header protrusion 22 and the inner protrusion 50 are welded together at a plurality of points (welds 60 and 61), the stress applied to the weld point that is positioned closest to the fluid inside the hollow fiber membrane module (weld 60) is alleviated, whereby the pressure capacity is improved and leakage of process fluid due to a failure in the weld point can be prevented. When the fluid goes out through between the potted part sectional surface 4S and the inner face of the header 5 as shown in Fig. 6 in the hollow fiber membrane module 1 under the internal pressure, the fluid pressure acts directly on the surfaces in contact with the fluid including the first weld 60 circumferentially continuously formed between the header protrusion 22 and the inner protrusion 50, so that the first weld 60 is subjected to shear stress (in the downward direction as indicated by the arrow in Fig. 6), which could speed up destruction of the weld. When the fluid pressure inside the header 5 builds up further and causes partial failure of the first weld 60, this pressure will act on the surfaces in contact with the fluid including the second weld 61 between the header protrusion 22 and the inner protrusion 50, so that the second weld is similarly subjected to shear stress (in the downward direction as indicated by the arrow in Fig. 6), which could speed up destruction of the weld. On the other hand, the third weld 62 between the header protrusion 22 and the outer protrusion 51 is capable of constantly creating a force that acts against the force caused by the internal pressure of the header 5 to move the header 5 away from the tubular container 2, during a period in which it does not contact the fluid directly, i.e., during the period in which no partial failure occurs in at least one of the first weld 60 and the second weld 61 circumferentially continuously formed. Thus, from the above viewpoint, too, the formation of the welds 60, 61, and 62 makes it hard for the header 5 to separate from the tubular container 2, and enables an increase in the pressure capacity of the hollow fiber membrane module 1.

Since the inner protrusion 50 and outer protrusion 51 of the tubular container 2 sandwich and hold the header protrusion 22 from both sides, radially inward or outward deflection of the header protrusion 22 can be minimized. This will minimize radial deflection of the header protrusion 22 even when the internal pressure of the hollow fiber membrane module 1 rises. Thus the possibility of separation of the weld interfaces between the header 5 and the tubular container 2 that can occur with deformation of the welds 60, 61, and 62 can be minimized. This all results in an increase in the pressure capacity of the hollow fiber membrane module 1.

According to this embodiment, the third weld 62 between the header protrusion 22 and the outer protrusion 51 is substantially at the same position in the longitudinal direction of the tubular container 2 as the second weld 61 between the header protrusion 22 and the inner protrusion 50, which can enhance the production efficiency of the welding process. Also, the second weld 61 between the header protrusion 22 and the inner protrusion 50 and the third weld 62 between the header protrusion 22 and the outer protrusion 51 can be formed at the same time during the ultrasonic welding process.

Preferably, the welds 60 and 61 between the header protrusion 22 and the inner protrusion 50 are formed in a plurality of different positions along the longitudinal direction of the tubular container 2, and there is a region (space 70) where the header 5 and the tubular container 2 do not contact each other between the different welds 60 and 61. Making the welds 60 and 61 independent from each other can prevent a crack failure of the welds, as a result of which possible destruction of weld points located closer to the fluid inside the hollow fiber membrane module (welds 60 and 61) occurs stepwise, so that the time until a weld failure can be extended. Furthermore, the region where the header 5 and the tubular container 2 do not contact each other, between at least one or more regions (welds 60 and 61) between the header protrusion 22 and the inner protrusion 50 and at least one or more regions (welds 62) between the header protrusion 22 and the outer protrusion 51, can prevent leakage of the process fluid inside the hollow fiber membrane module 1 out of the system, which may result from a weld crack failure.

Since the inner protrusion 50 of the tubular container 2 and the header protrusion 22 are welded together at two regions, and the outer protrusion 51 of the tubular container 2 and the header protrusion 22 are welded together at one region, the process fluid that has just leaked out inside the header protrusion 22 can be stopped sufficiently twice, so that, even if the process fluid passes through inside the header protrusion 22, the process fluid can be stopped from leaking out of the header protrusion 22. Thus welding at two inner locations and one outer location can significantly increase the pressure capacity.

The strength of each of the welds 60 to 62 can be increased further by being formed circumferentially continuously all around the header 5.

End faces of the potted parts 4 at both ends are formed as potted part sectional surfaces 4S by radially cutting the potted parts at both ends, and the potted part sectional surfaces 4S are in tight contact with the flat surfaces 40 of the inner faces of the headers 5. This prevents the fluid from readily flowing into a gap between the header protrusion 22 and the end part 2a of the tubular container 2, and improves the seal between the header 5 and the tubular container 2.

While there are welds at two locations between the header protrusion 22 and the inner protrusion 50 and at one location between the header protrusion 22 and the outer protrusion 51 in the embodiment described above, the number of respective welds is not limited to this and can be selected as required. The welds at some locations may be formed with a shear joint design.

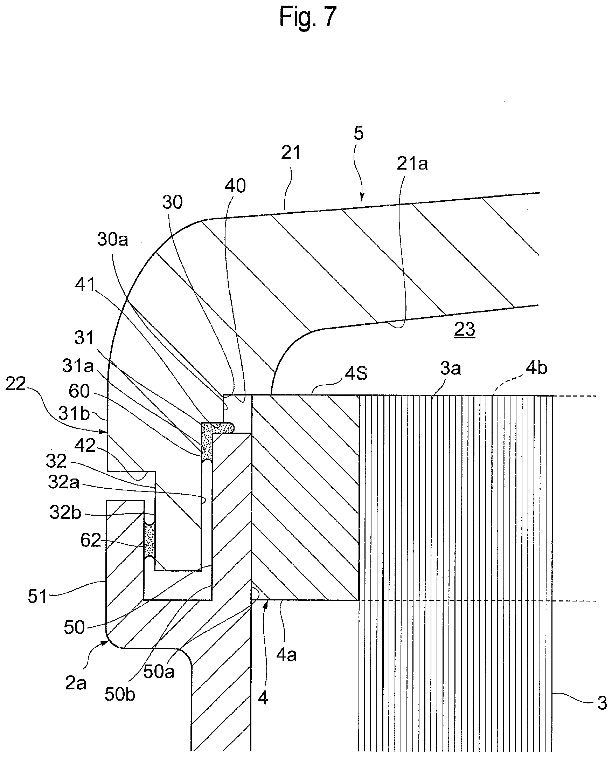

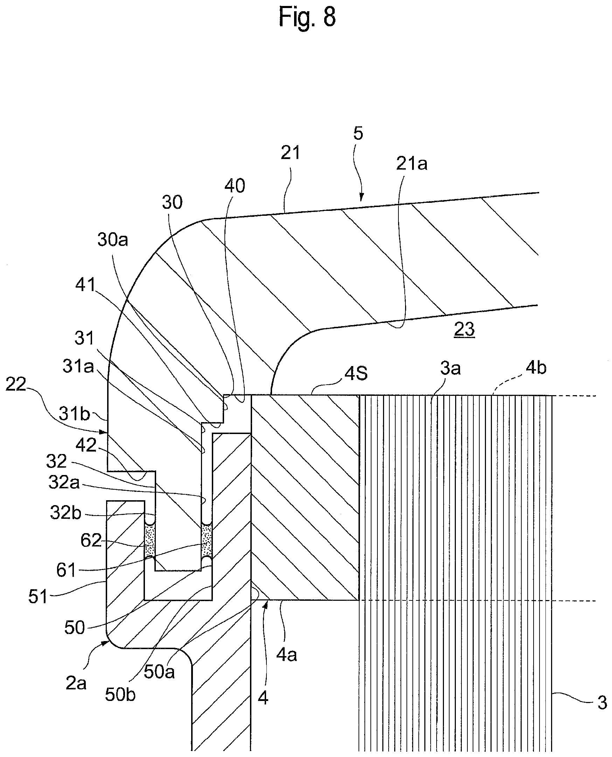

For example, there may be only the first weld 60 and the third weld 62 as shown in Fig. 7, or there may be only the second weld 61 and the third weld 62 as shown in Fig. 8. There may be welds at three or more locations between the header protrusion 22 and the inner protrusion 50, and there may be welds at two or more locations between the header protrusion 22 and the outer protrusion 51.

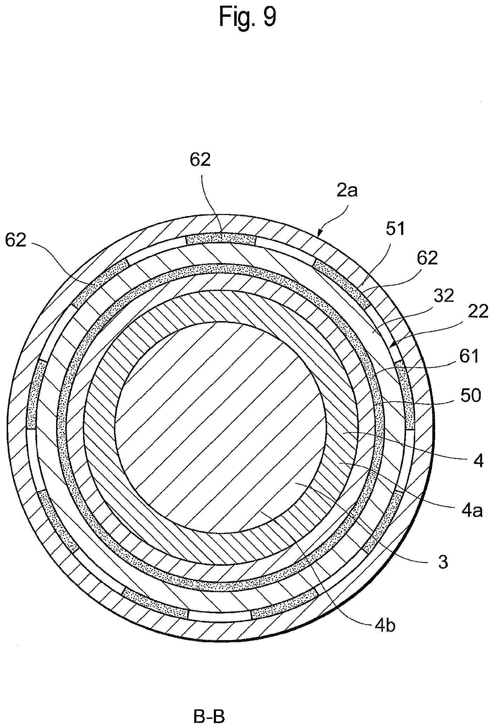

While the welds 60 to 62 are circumferentially continuously formed all around the header 5 in the embodiment described above, they may be circumferentially intermittently formed around the header 5. For example, as shown in Fig. 9, only the third weld 62 on the outer side of the header protrusion 22 may be circumferentially intermittently formed around the header 5. Alternatively, at least one of the welds 60 and 61 may be circumferentially intermittently formed around the header 5.

While a preferred embodiment of the present invention has been described above with reference to the accompanying drawings, the present invention is not limited to this example. It is understood that a person skilled in the art could obviously conceive various examples of changes or modifications within the scope of the idea recited in the claims and that these shall also naturally belong to the technical scope of the present invention.

For example, the structure of the header protrusion 22 of the header 5, or the structures of the inner protrusion 50 and outer protrusion 51 of the tubular container 2, of the embodiment described above, are not limited to those shown. For example, the header protrusion 22 of the header 5, or the inner protrusion 50 and outer protrusion 51 of the tubular container 2, may be intermittently formed at some locations along the circumferential direction of the hollow fiber membrane module 1, or only in some parts of the module, instead of being formed in an annular shape all around the hollow fiber membrane module 1. The entire structures of the tubular container 2 and header 5 are also not limited to those shown. The purpose of use of the hollow fiber membrane module 1 is not limited to processing of fluids such as blood and it may be used for processing of gasses.

### Examples

Hereinafter, specific examples and comparative examples will be described, but the present invention shall not be limited to the following examples.

### <Cross-sectional weld area>

X-ray CT measurement was made to hollow fiber membrane modules obtained in various examples and comparative examples to be described below, to determine the cross-sectional areas (total sum of weld areas) of parts where the header and the tubular container were fused or overlapping each other as a result of the interference parts disposed on the header or tubular container interfering each other, in an observation cross section passing through the center axis P of the hollow fiber membrane module 1.

### <Pressure capacity evaluation: Pressure capacity test>

A pressure capacity test was carried out in the following procedure. A drill was inserted from the nozzle part of the header of the hollow fiber membrane modules obtained in various examples and comparative examples to be described below to form a through hole in part of the potting resin fixed part. Water was poured from the nozzle part of the header and the port of the tubular container, and plugs were attached to two points in the nozzle part of the header and to the port of the tubular container. After that, water was injected from the port of the tubular container using a hydraulic pump to increase the pressure by 0.1 MPa each, and the pressure was measured until a failure was observed in welds or hollow fiber membrane modules. Pressure was increased until it reached a maximum of 2.0 MPa.

### [Example 1]

A hollow fiber membrane module 1 such as the one shown in Fig. 2 was fabricated. Polypropylene resin was used for the molding of the headers 5 and tubular container 2. An interference part with a shear joint design was provided all around the inner circumferential surface 30a of the base part 30 of the header 5 so as to form the first weld 60. An interference part with a shear joint design was provided all around the outer circumferential surface 50b of the inner protrusion 50 of the tubular container 2 so as to form the second weld 61. An interference part with a shear joint design was provided all around the inner circumferential surface 51a of the outer protrusion 51 of the tubular container 2 so as to form the third weld 62.

The inner circumferential surface 50a of both end parts of the tubular container 2 was subjected to an atmospheric corona treatment, after which a hollow fiber membrane bundle 3, which is a bundle of 7,700 hollow fiber membranes having an inner diameter of 200 µm and a membrane thickness of 43 µm, was inserted. After injection of potting resin, centrifugal rotation, and storage for curing, the hollow fiber membrane bundle 3 was embedded and retained at both end parts of the tubular container 2 to form potting resin fixed parts 4. The potting resin fixed parts 4 protruding from both end parts of the tubular container 2 were uniformly cut all around to open the end parts of the hollow fiber membrane bundle 3. The average height of the sectional surfaces 4S of the potting resin fixed parts at this time was 0.7 mm.

The headers 5 were assembled to the tubular container 2 with the potting resin fixed parts 4 having been cut as described above, and after it was confirmed that the header protrusion 22 was inserted between the inner protrusion 50 and outer protrusion 51 of the tubular container 2, ultrasonic welding was carried out under conditions with a frequency of 20 kHz, pressure of 0.3 MPa, welding time of 0.15 sec, and holding time of 0.5 sec.

Using the hollow fiber membrane modules 1 thus obtained, the pressure capacity test mentioned above was conducted. Neither the welds 60 to 62 nor the hollow fiber membrane modules 1 of Example 1 in which the first weld 60, second weld 61, and third weld 62 were all formed circumferentially continuously failed, even when subjected to a maximum pressure of 2.0 MPa.

### [Examples 2 to 6]

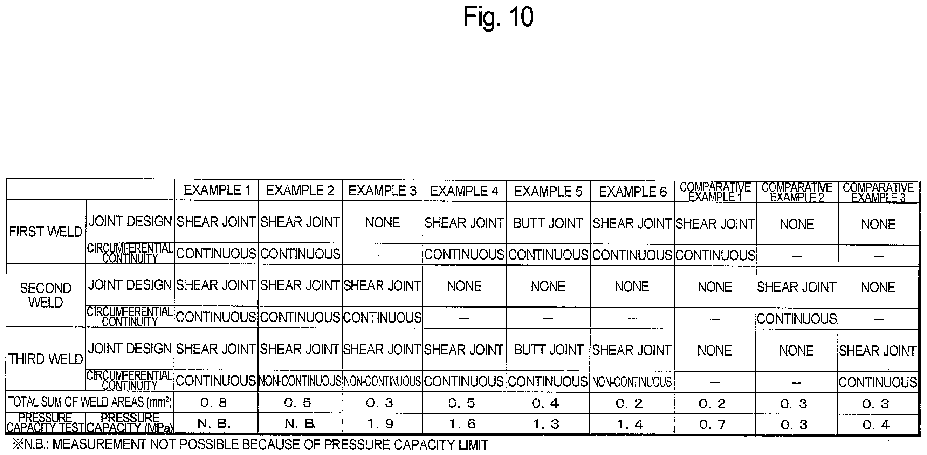

Hollow fiber membrane modules 1 were fabricated similarly to Example 1 except for differences in the presence/absence of the first to third welds and the circumferential continuity and non-continuity of each weld as specified in Fig. 10, and the pressure capacity test was conducted to these hollow fiber membrane modules 1. The results of evaluation of these hollow fiber membrane modules 1 are shown in Fig. 10.

### [Comparative example 1]

A hollow fiber membrane module 1 was fabricated similarly to Example 1 except that no interference part was provided to the inner protrusion 50 and outer protrusion 51 of the tubular container 2, i.e., the second weld and third weld were not provided, and the pressure capacity test was conducted to this hollow fiber membrane module 1. The results of evaluation of this hollow fiber membrane module 1 are shown in Fig. 10.

### [Comparative example 2]

A hollow fiber membrane module 1 was fabricated similarly to Example 1 except that no interference part was provided to the inner circumferential surface 30a of the base part 30 of the header 5 and no interference part was provided to the outer protrusion 51 of the tubular container 2, i.e., the first weld and third weld were not provided, and the pressure capacity test was conducted to this hollow fiber membrane module 1. The results of evaluation of this hollow fiber membrane module 1 are shown in Fig. 10.

### [Comparative example 3]

A hollow fiber membrane module 1 was fabricated similarly to Example 1 except that no interference part was provided to the inner circumferential surface 30a of the base part 30 of the header 5 and no interference part was provided to the inner protrusion 50 of the tubular container 2, i.e., the first weld and second weld were not provided, and the pressure capacity test was conducted to this hollow fiber membrane module 1. The results of evaluation of this hollow fiber membrane module 1 are shown in Fig. 10.

### Industrial Applicability

The present invention is useful for increasing the pressure capacity of hollow fiber membrane modules.

### Reference Signs List

- 1: Hollow fiber membrane module
- 2: Tubular container
- 2a: End part
- 3: Hollow fiber membrane bundle
- 4: Potted part
- 4S: Potted part sectional surface
- 5: Header
- 22: Header protrusion
- 50: Inner protrusion
- 51: Outer protrusion
- 60: First weld
- 61: Second weld
- 62: Third weld

## Claims

1. A hollow fiber membrane module comprising:
a tubular container accommodating a hollow fiber membrane bundle;
a header provided to each of both end parts of the tubular container and having an outlet/inlet port of a fluid; and
a potted part retaining fixedly the hollow fiber membrane bundle embedded therein at each of both end parts of the tubular container,
the header having a header protrusion protruding toward the tubular container,
the tubular container having double protrusions, which are an inner protrusion and an outer protrusion protruding toward the header,
the header and the tubular container being welded together by ultrasonic welding, with the header protrusion being inserted between the inner protrusion and the outer protrusion of the tubular container, and
the header protrusion and the inner protrusion of the tubular container being welded together at least in one or more regions, and the header protrusion and the outer protrusion of the tubular container being welded together at least in one or more regions.

2. The hollow fiber membrane module according to claim 1, wherein at least one portion of a weld between the header protrusion and the outer protrusion of the tubular container, and at least one portion of a weld between the header protrusion and the inner protrusion of the tubular container, are located substantially at the same position in a longitudinal direction of the tubular container.

3. The hollow fiber membrane module according to claim 1 or 2, wherein a weld is formed between the header protrusion and the inner protrusion of the tubular container at a plurality of different locations in the longitudinal direction of the tubular container, and a region where the header and the tubular container do not contact each other exists between the welds at different locations.

4. The hollow fiber membrane module according to any one of claims 1 to 3, wherein a region where the header and the tubular container do not contact each other exists between a weld between the header protrusion and the inner protrusion of the tubular container and a weld between the header protrusion and the outer protrusion of the tubular container.

5. The hollow fiber membrane module according to any one of claims 1 to 4, wherein at least a portion of the weld between the header protrusion and the inner protrusion of the tubular container, or, at least a portion of the weld between the header protrusion and the outer protrusion of the tubular container, is circumferentially continuously formed all around the header.

6. The hollow fiber membrane module according to any one of claims 1 to 5, wherein the inner protrusion of the tubular container and the header protrusion are welded together at two regions, while the outer protrusion of the tubular container and the header protrusion are welded together at one region.

7. The hollow fiber membrane module according to any one of claims 1 to 6, wherein
an end face of the potted part at each of the both ends is formed to be a potted part sectional surface formed by radially cutting the potted part at each of the both ends, and
the potted part sectional surface is in contact with an inner side face of the header.

8. The hollow fiber membrane module according to any one of claims 1 to 7, wherein the header and the tubular container are made of polypropylene resin.

9. A method of producing hollow fiber membrane modules including a tubular container accommodating a hollow fiber membrane bundle, a header provided to each of both end parts of the tubular container and having an outlet/inlet port of a fluid, and a potted part retaining fixedly the hollow fiber membrane bundle embedded therein at each of the both end parts of the tubular container,
the method comprising the steps of:
forming the header such that the header has a header protrusion protruding toward the tubular container, this tubular container having double protrusions, which are an inner protrusion and an outer protrusion protruding toward the header; and
welding the header and the tubular container together by ultrasonic welding, with the header protrusion being inserted between the inner protrusion of the tubular container and the outer protrusion of the tubular container, wherein
in the welding step, the header protrusion and the inner protrusion of the tubular container are welded together at least in one or more regions, and the header protrusion and the outer protrusion of the tubular container are welded together at least in one or more regions.
